# EUROPÄISCHE PATENTSCHRIFT

(11) **EP 0 701 559 B1**
(45) Veröffentlichungstag und Bekanntmachung des Hinweises auf die Patenterteilung: **29.07.1998**
(21) Anmeldenummer: 94919619.0
(22) Anmeldetag: 03.06.1994
(51) Int. Cl.: C07D 491/22, A61K 31/495

(54) **CEPHALOSTATIN-ANALOGA UND IHRE VERWENDUNG ALS ANTITUMORMITTEL**
CEPHALOSTATIN ANALOGUES AND THEIR USE AS ANTI-TUMOUR AGENTS
ANALOGUES DE CEPHALOSTATINE ET LEUR UTILISATION COMME AGENTS ANTITUMORAUX

(30) Priorität: 03.06.1993 DE 4318924
(43) Veröffentlichungstag der Anmeldung: 20.03.1996
(73) Patentinhaber: SCHERING AKTIENGESELLSCHAFT, 13353 Berlin (DE)
(72) Erfinder: WINTERFELDT, Ekkehard, D-30916 Isernhagen (DE); KRAMER, Andreas, D-30419 Hannover (DE); ULLMANN, Ulrike, D-30167 Hannover (DE); LAURENT, Henry, D-13467 Berlin (DE)
(86) Internationale Anmeldenummer: EP9401858
(87) Internationale Veröffentlichungsnummer: WO9429318

(56) Entgegenhaltungen:
- JOURNAL OF ORGANIC CHEMISTRY. Bd. 57, Nr. 2 , 17. Januar 1992 , EASTON US Seiten 429 - 431 G. R. PETTIT ET AL 'Antineoplastic agents. 214. Isolation and structure of cephalostatins 7-9.' in der Anmeldung erwähnt
- JOURNAL OF THE CHEMICAL SOCIETY, PERKIN TRANSACTIONS 1 Nr. 23 , 7. Dezember 1993 , LETCHWORTH GB Seiten 2865 - 2867 A. KRAMER ET AL 'A short route to cephalostatin analogues'
- BIOORG. MED. CHEM. LETT. (1992) 2(9), 967-972
- J. ORG. CHEM. (1992) 57(24), 6379-6380
- J. ORG. CHEM. (1994) 59(22), 6829-6839

## Beschreibung

Die vorliegende Erfindung betrifft Cephalostatin-Analoga der allgemeinen Formel I, 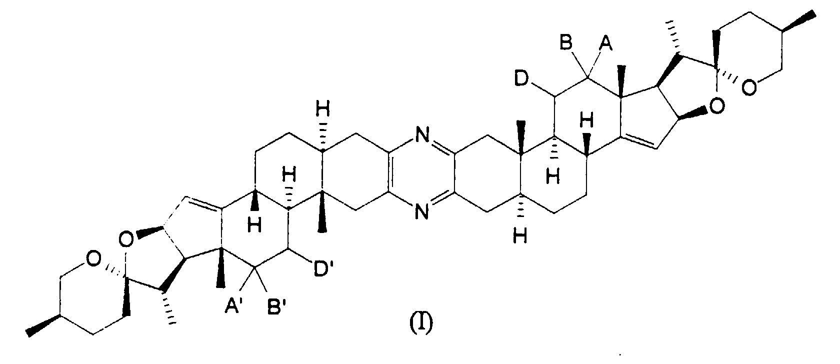 worin
a) A und A' je für eine Alkanoyloxygruppe R-CO-O- mit R als gerad- oder verzweigtkettigen C₁- bis C₇-Alkylrest und B und D sowie B' und D' je gemeinsam für eine zusätzliche C-C-Bindung oder
b) A' für eine Alkanoyloxygruppe R-CO-O- mit R als gerad- oder verzweigtkettigen C₁- bis C₇-Alkylrest und B' und D' gemeinsam für eine zusätzliche C-C-Bindung sowie A und B gemeinsam für ein Ketosauerstoffatom und D für ein Wasserstoffatom oder
c) A für eine β-ständige Hydroxygruppe und B sowie D je für ein Wasserstoffatom sowie A' und B' gemeinsam für ein Ketosauerstoffatom und D' für ein Wasserstoffatom oder
d) A und A' je für eine β-ständige Hydroxygruppe und B, D, B' und D' je für ein Wasserstoffatom
stehen,
ein Verfahren zur Herstellung dieser Cephalostatin-Analoga, diese Cephalostatin-Analoga enthaltende Arzneimittel sowie deren Verwendung zur Herstellung von Arzneimitteln.

Die Cephalostatine stellen eine Gruppe komplexer steroidaler Pyrazinalkaloide dar, die aus dem Meereswurm *Cephalodiscus gilchristi* ((a) Pettit, G.R.; Kamano, Y.; Dufresne, C.; Inoue, M.; Christi, N.; Schmidt, J.M.; Doubek, D.L.; Can. J. Chem. 1989, 67, 1509. (b) Pettit, G.R.; Inoue, M.; Kamano, Y.; Herald, D.L.; Arm, C.; Dufresne, C.; Christie, N.D.; Schmidt, J.M. Doubek, D.L.; Krupa, T.S.; J. Am. Chem. Soc. 1988, 110, 2006. (c) Pettit, G.R.; Inoue, M.; Kamano, Y.; Dufresne, C.; Christie, N.D.; Niven, M.L.; Herald, D.L.; J. Chem. Soc., Chem. Commun. 1988, 865. (d) Pettit, G.R.; Kamano, Y.; Inoue, M.; Dufresne, C.; Boyd, R.; Herald, D.L.; Schmidt, J.M.; Doubek, D.L.; Christie, N.D.; J. Org. Chem. 1992, 57, 429.) isoliert wurden. Sie stellen hochwirksame Cytotoxine gegen die PS-Zell-Linie (ED₅₀ 10⁻⁷ - 10⁻⁹ µg/ml) dar und besitzen ein potentielles Interesse als Antitumormittel. Sie sind jedoch selten vorkommende marine Naturprodukte und sind nur in äußerst geringen Mengen verfügbar. Beispielsweise konnten aus 166 kg *Cephalodiscus gilchristi* (5 mm lange röhrchenförmige Würmer) lediglich 139 mg Cephalostatin 1 und eine Gesamtmenge von 272 mg anderer Cephalostatine isoliert werden.

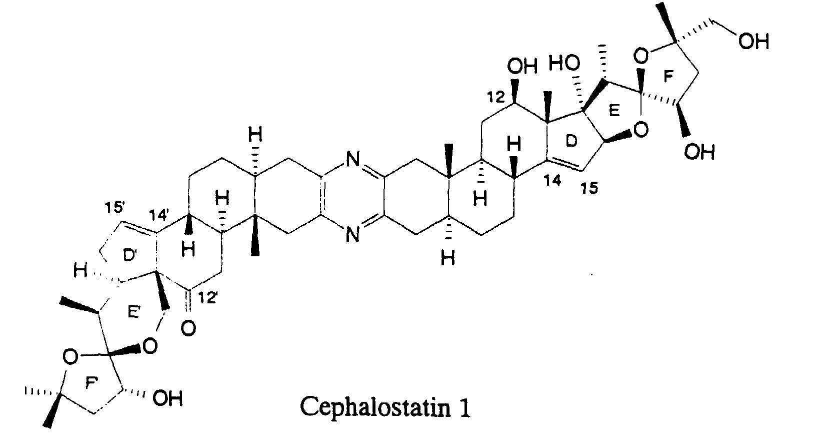

Das Cephalostatin 1 stellt ein asymmetrisch aufgebautes und substituiertes Bis-Steroidopyrazin dar, das im D- und D'-Ring jeweils eine Doppelbindung aufweist.

Beim erst kürzlich bekannt gewordenen Cephalostatin 7 (Pettit, G.R. et al., loc. cit. (d)) hingegen handelt es sich um ein bezüglich des Grundkörpers symmetrisch aufgebautes Bis-Steroidopyrazin, das sich lediglich in seinem F- und F'-Ring unterscheidet.

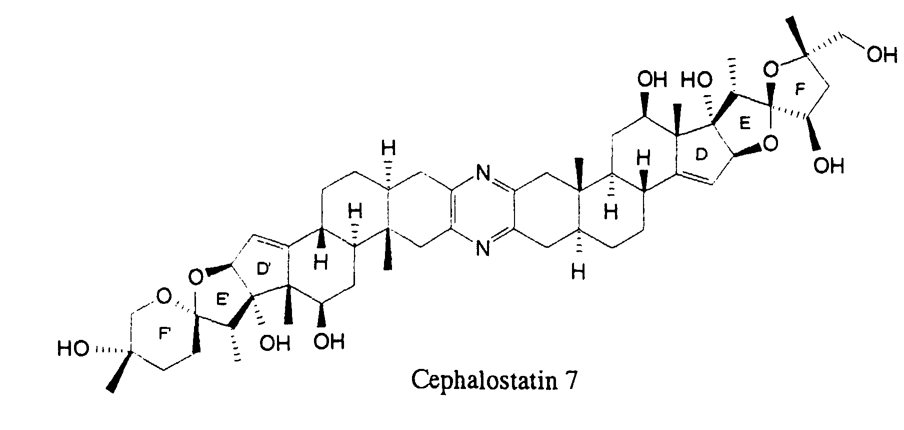

Obwohl die Cephalostatine mit zu den potentesten Cytotoxinen gehören, die je vom National Cancer Institute (USA) im PS-System gescreent wurden, sind In-vivo-Tests aufgrund der beschränkten Verfügbarkeit der natürlichen Materialien limitiert (S.C. Smith and C.H. Heathcock, J. Org. Chem. 1992, 57, 6379). Wegen der beschränkten Verfügbarkeit besteht aber reges Interesse, eine Totalsynthese für Cephalostatine zu verwirklichen. Von S.C. Smith and C.H. Heathcock (loc. cit.) werden drei Verfahren zur Bildung von Bis-Steroidopyrazinen beschrieben: zwei dieser Verfahren sind zur Herstellung von symmetrischen Bis-Steroidopyrazinen in hohen Ausbeuten anwendbar, während das dritte Verfahren die Synthese bisher lediglich in Form der natürlich vorkommenden Cephalostatine bekannten asymmetrischer Analoga ermöglicht.

Die gemäß der erwähnten Literaturstelle herstellbaren symmetrisch (R = C₈H₁₇) oder asymmetrisch (R = OAc) aufgebauten Cephalostatin-Analoga sind vom nachstehend gezeigten Typ:

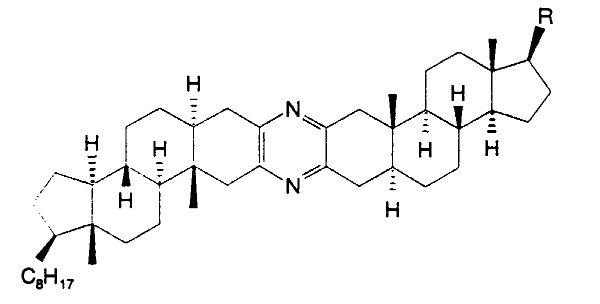

Abgesehen von der stark abweichenden D-Ring-Substitution weisen diese Cephalostatin-Analoga außerdem weder die für das Cephalostatin 1 und Cephalostatin 7 typischen 12-Hydroxy- und 12'-Keto- bzw. 12'-Hydroxyfunktionen noch die dort in dem D- und D'-Ring vorkommenden Doppelbindungen auf. Diese Doppelbindungen sind von Bedeutung für eine erhöhte Löslichkeit derartiger Verbindungen.

Aufgabe der vorliegenden Erfindung ist es, den natürlich vorkommenden Cephalostatinen, insbesondere den Cephalostatinen 1 und 7, strukturell möglichst nahekommende Cephalostatin-Analoga zur Verfügung zu stellen, die außerdem nach einem einfachen Verfahren, ausgehend von einem leicht zugänglichen Ausgangsprodukt, herstellbar sein sollen.

Diese Aufgabe wird durch die Bereitstellung der Verbindungen der allgemeinen Formel I, 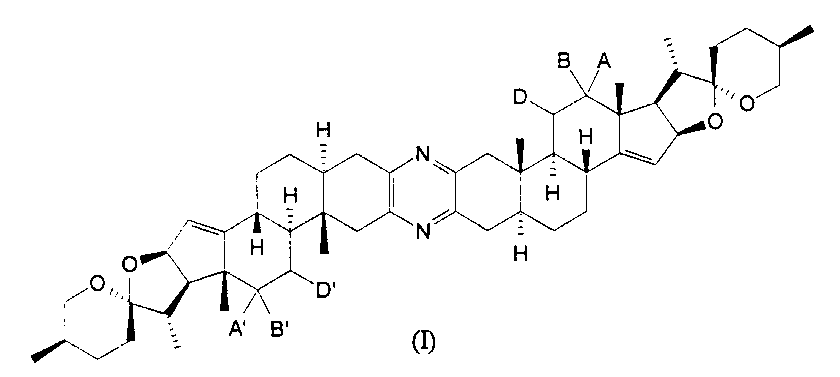 worin
a) A und A' je für eine Alkanoyloxygruppe R-CO-O- mit R als gerad- oder verzweigtkettigen C₁- bis C₇-Alkylrest und B und D sowie B' und D' je gemeinsam für eine zusätzliche C-C-Bindung oder
b) A' für eine Alkanoyloxygruppe R-CO-O- mit R als gerad- oder verzweigtkettigen C₁- bis C₇-Alkylrest und B' und D' gemeinsam für eine zusätzliche C-C-Bindung sowie A und B gemeinsam für ein Ketosauerstoffatom und D für ein Wasserstoffatom oder
c) A für eine β-ständige Hydroxygruppe und B sowie D je für ein Wasserstoffatom sowie A' und B' gemeinsam für ein Ketosauerstoffatom und D' für ein Wasserstoffatom oder
d) A und A' je für eine β-ständige Hydroxygruppe und B, D, B' und D' je für ein Wasserstoffatom
stehen,
gelöst.
R-CO- bedeutet beispielsweise einen Acetyl-, Propionyl-, n-, iso- oder tert.-Butyryl-, Pivaloyl- oder Valeryl- oder einen höheren Alkanoylrest. Ein Pivaloylrest ist hierfür bevorzugt.

Die Fälle Ic (12β-Hydroxy-bis[(25R)-5α-spirost-14-eno][2,3-b;2',3'-e]pyrazin-12'-on) und Id (Bis[(25R)-5α-spirost-14-eno][2,3-b;2',3'-e]pyrazin-12β,12β'-diol) sind bevorzugt.

Die Verbindungen der Formel I weisen entweder wie das Cephalostatin 1 eine 12-Hydroxy- und eine 12'-Ketofunktion oder wie das Cephalostatin 7 eine 12- und 12'-Hydroxygruppe sowie eine 14,15- und 14',15'-Doppelbindung im D- bzw. D'-Ring auf, die eine gute Löslichkeit der entsprechenden Verbindungen gewährleistet. Das Substitutionsmuster des D- und D'-Ringes entspricht zwar nicht exakt demjenigen des Cephalostatins 1 oder des Cephalostatins 7, doch sind auch diese beiden Ringe wie im Cephalostatin 1 und insbesondere im Cephalostatin 7 jeweils durch ein ankondensiertes Spirosystem mit je einem Sauerstoffatom in jedem Ring der Spirostrukturelemente substituiert.
Es wurde nunmehr gefunden, daß die erfindungsgemäßen Verbindungen der allgemeinen Formel I wie das Cephalostatin 1 hochwirksame, in verschiedenen menschlichen Zell-Linien das Zellwachstum inhibierende Verbindungen sind.

Aufgrund der cytotoxischen Potenz können die erfindungsgemäßen Verbindungen zur Herstellung von Arzneimitteln zur Behandlung der verschiedensten Tumorarten verwendet werden.
Die vorliegende Erfindung betrifft somit auch die Verwendung der erfindungsgemäßen Verbindungen zur Herstellung von Arzneimitteln, insbesondere zur Behandlung und Bekämpfung von Carcinomen, Sarkomen und Leukämien.

Des weiteren betrifft die vorliegende Erfindung pharmazeutische Präparate, die mindestens eine Verbindung der allgemeinen Formel I sowie einen pharmazeutisch verträglichen Träger enthalten.

Die zu verabreichende Menge der Verbindung(en) schwankt innerhalb eines weiten Bereiches und kann jede wirksame Menge abdecken. In Abhängigkeit des zu behandelnden Zustands und der Art der Verabreichung kann die Menge der verabreichten Verbindung 0,1 - 50 mg/kg Körpergewicht, vorzugsweise 1 - 30 mg/kg Körpergewicht, je Tag betragen.

Zur oralen Verabreichung kommen Kapseln, Pillen, Tabletten, Dragees usw. infrage. Die Dosierungseinheiten können neben dem Wirkstoff einen pharmazeutisch verträglichen Träger, wie z.B. Stärke, Zucker, Sorbit, Gelatine, Gleitmittel, Kieselsäure, Talkum usw. enthalten. Die einzelnen Dosierungseinheiten für die orale Applikation können beispielsweise 1 bis 100 mg des Wirkstoffes enthalten.

Zur intravenösen Gabe der erfindungsgemäßen Verbindungen kommen deren isotonische wäßrige Lösungen infrage.

Die subcutane Applikation der erfindungsgemäßen Verbindungen ist in einem Lösungsmittel wie Wasser und/oder Polyethylenglycol möglich.

Die Formulierung und Applikation der erfindungsgemäßen Verbindungen kann analog wie bei den bekannten Cytostatika Etoposid oder Methotrexat vorgenommen werden.

Die neuen Verbindungen der allgemeinen Formel I werden hergestellt, indem die Verbindung der Formel II, 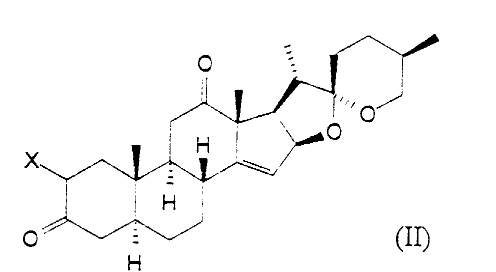 worin X für ein Bromatom steht, durch Umsetzung mit einem Alkaliazid AlkaliN₃, worin Alkali für ein Lithium-, Natrium- oder Kaliumatom steht, in die Verbindung der Formel II, worin X für die Azidgruppe -N₃ steht, überführt,
dieses α-Ketoazid unter Erwärmen und Stickstoffverlust zum 2-Imino-3-keton der Formel III 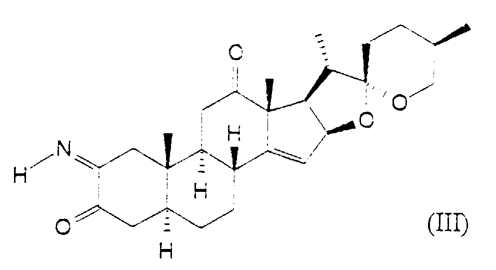 abgebaut,
dieses unter Bedingungen einer katalytischen Hydrierung zum symmetrischen Pyrazindiketon der Formel IV 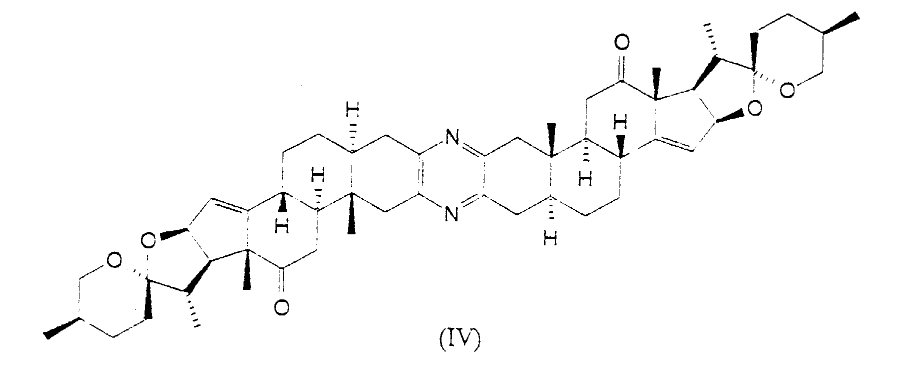 dimerisiert,
und, wenn eine Verbindung der allgemeinen Formel I mit der Substituentendefinition a), b) oder d) hergestellt werden soll,
a) dieses Bis-12,12'-keto-steroidopyrazin durch Überführung beider Ketogruppen in das Enolat und dessen Abfang als C₂- bis C₈-Carbonsäureester mit einem Carbonsäurechlorid oder -bromid oder einem Carbonsäureanhydrid der allgemeinen Formel V,

   R-CO-Y bzw. (R-CO)₂O (V)

   worin
   R einen gerad- oder verzweigtkettigen C₁- bis C₇-Alkylrest und
   Y ein Chlor- oder Bromatom
   darstellen,
   in einen Diester der allgemeinen Formel Ia, 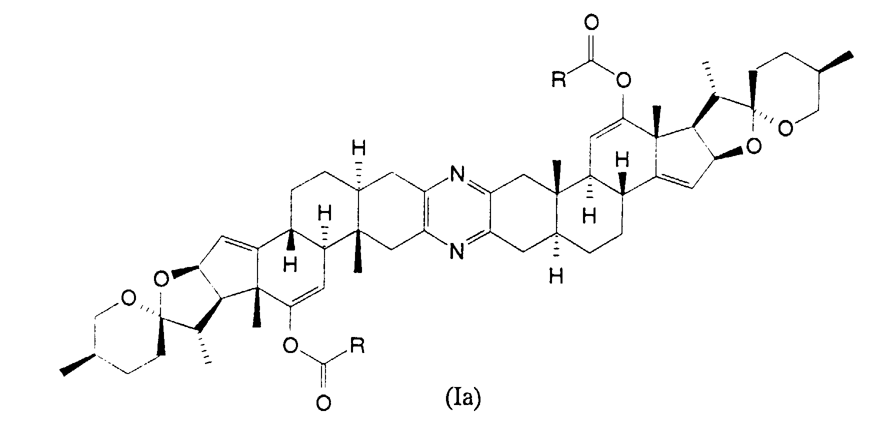 worin R einen gerad- oder verzweigtkettigen C₁-C₇-Alkylrest bedeutet,
   überführt oder
b) dieses Bis-12,12'-keto-steroidopyrazin durch Überführung einer der Ketogruppen in das Enolat und dessen Abfang mit einer Verbindung der allgemeinen Formel V als C₂- bis C₈-Carbonsäureester zu einem Monoester der allgemeinen Formel Ib, 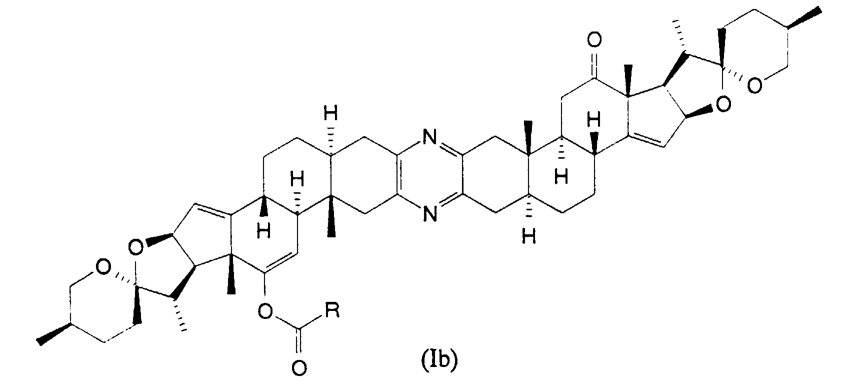 worin R einen gerad- oder verzweigtkettigen C₁- bis C₇-Alkylrest bedeutet, umgesetzt, und gegebenenfalls
c) aus der 12-Keto-12'-alkanoyloxy-Verbindung der allgemeinen Formel Ib durch Reduktion der 12-Ketogruppe mit einem Alkali- oder Ammoniumborhydrid (Alkali = Lithium, Natrium, Kalium; Ammonium = NH₄⁺ oder NAlkyl₄⁺, Alkyl = C₁-C₄) und Verseifung (und Tautomerisierung) der 12'-Estergruppe in die Verbindung der Formel Ic 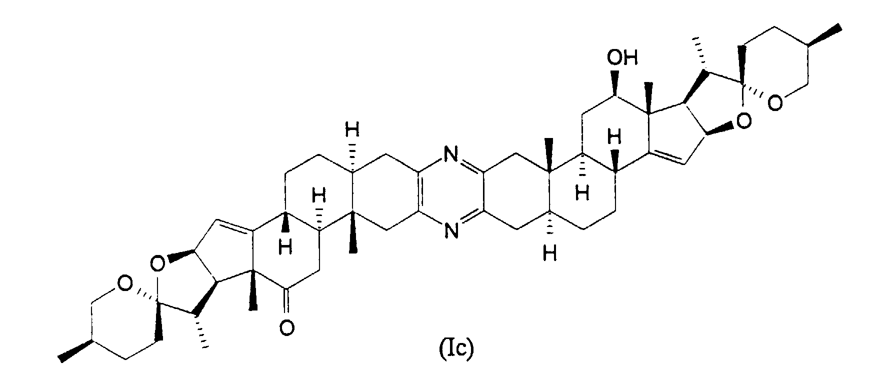 umgewandelt oder
d) dieses Bis-12,12'-keto-steroidopyrazin mit einem Alkali- oder Ammoniumborhydrid (Alkali und Ammonium vgl. Ic) zum Bis-12,12'-hydroxy-steroidopyrazin der allgemeinen Formel Id 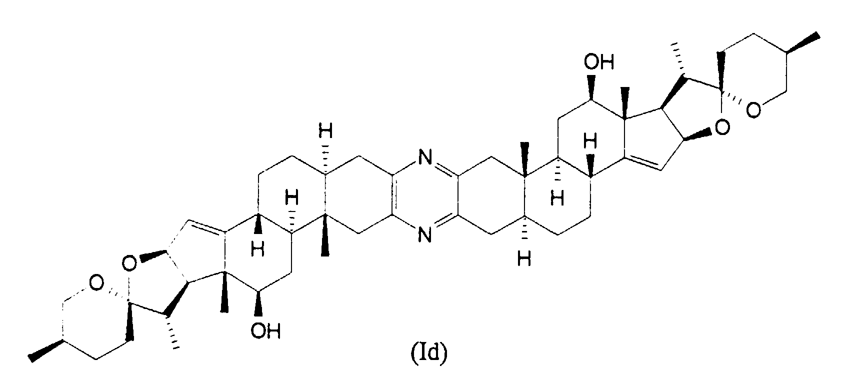 reduziert wird.

Das beschriebene Verfahren umfaßt eine einfach durchzuführende symmetrische Pyrazinsynthese; die unter Umständen letztendlich gewünschte Asymmetrie (Verbindung der allgemeinen Formel Ib oder Ic) wird erst auf einer späteren Reaktionsstufe eingeführt.

Als Startverbindung des beschriebenen Verfahrens dient das Δ¹⁴-Hecogeninderivat der Formel II, worin X ein Bromatom darstellt. Dieses ist leicht zugänglich aus dem reichlich zur Verfügung stehenden Hecogenin 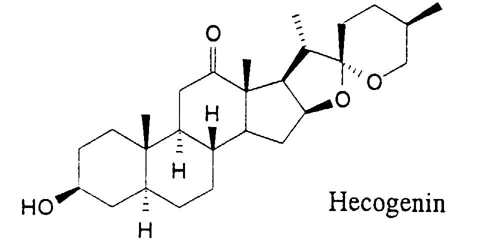 und zwar durch ein etabliertes Bestrahlungsverfahren mit anschließender Oxidation und Bromierung in 2-Position nach Standardverfahren (P. Welzel, B. Janssen, H. Dudek, Liebigs Ann. Chem. 1981, 546).
Die sich anschließende Umsetzung mit Lithium-, Natrium- oder Kaliumazid, vorzugsweise Natriumazid (nucleophile Substitution), wird in einem polaren Lösungsmittel wie Dimethylformamid, Dimethylsulfoxid oder Isopropanol, vorzugsweise unter der katalytischen Einwirkung eines Alkalijodids wie etwa Natriumjodid, bei einer Temperatur von 0 bis 100°C, beispielsweise 50 °C, durchgeführt.
Nach der Aufarbeitung mit Lauge wird in sehr guter Ausbeute (bis 90 %) direkt das Iminoketon III erhalten, das als solches nicht zur Pyrazinbildung neigt. Unter den Bedingungen einer katalytischen Hydrierung hingegen (Palladium [10 %] auf Aktivkohle Hydrierung bei Raumtemperatur) bildet sich spontan der symmetrisch aufgebaute Vorläufer IV der letztendlich herzustellenden Cephalostatin-Analoga der allgemeinen Formel I. Als Lösungsmittel dient hier ein polares Solvens wie etwa Essigsäureethylester unter Zusatz einer geringen Menge eines Alkohols, beispielsweise Methanol. Die Reaktion wird dünnschichtchromatographisch verfolgt. Nach Aufarbeitung und Chromatographie wird das Pyrazin IV in ebenfalls guter Ausbeute von ca. 65 % erhalten.

Die vorliegende Erfindung betrifft auch das Pyrazindiketon der Formel IV als Zwischenprodukt

Aus dem Pyrazindiketon IV wird dann in einer Schutzgasatmosphäre durch Protonenabfang mit einem Überschuß Natrium- oder Kaliumhexamethyldisilazan das entsprechende Di- bzw. Monoenolat erzeugt, welches durch Abfang mit einem Carbonsäurechlorid oder -bromid oder einem Carbonsäureanhydrid der allgemeinen Formel V,

R-CO-Y bzw. (R-CO)₂O (V)

worin
R einen gerad- oder verzweigtkettigen C₁- bis C₇-Alkylrest und
Y ein Chlor- oder Bromatom
darstellen,
als Di- oder Monocarbonsäureester der allgemeinen Formel Ia bzw. Ib stabilisiert wird.

Die Enolate werden vorzugsweise mit Pivaloylchlorid abgefangen und als Pivalate stabilisiert.
Die Erzeugung und der Abfang der Enolate wird in trockener etherischer Lösung, beispielsweise in Tetrahydrofuran, vorgenommen.
Ob bevorzugt das Di- oder das Monoenolat gebildet wird, hängt u.a. von der Höhe des Überschusses des Enolisierungsmittels Natrium- oder Kaliumhexamethyldisilazan ab: Ein hoher Überschuß von über 4 Equivalenten führt in erster Linie und überwiegend zum Dienolat; nach Abfang beispielsweise als Pivalat wird ein Gemisch des in diesem Fall gewünschten Bis-enolpivalats Ia und des asymmetrischen Monopivalats Ib erhalten.

Bei Verwendung von 3 bis 3,5, vorzugsweise von 3,2 Äquivalenten des Enolisierungsmittels Kaliumhexamethyldisilazan und Abfang als Pivalat wird ein ca. 1:2 Gemisch des Bis-enolpivalats Ia und des gewünschten, asymmetrischen Mono-pivalats Ib erhalten. Deren Trennung gelingt in einfacher Weise durch Säulenchromatographie. Obwohl, bedingt durch die symmetrische Struktur des eingesetzten Pyrazindiketons, die Reaktion nicht ausschließlich zum Monoenolat und Monoenolester führen kann, wird so letztendlich der Monoenolester Ib immerhin in einer beachtlichen Ausbeute von ca. 40% erhalten.
Ob bevorzugt das Mono- oder Dienolat und der entsprechende Ester entsteht, hängt außer vom Basenüberschuß auch von der Reaktionszeit und der Menge des zum Abfang des gebildeten Enolats angebotenen Säurehalogenids (Pivaloylchlorid) oder Säureanhydrids ab. Aus dem Monoenolester Ib schließlich wird durch Borhydrid-Reduktion (Lithium-, Natrium-Kaliumboranat oder ein Ammoniumboranat) und nachfolgende Verseifung der Enolestergruppe und Tautomerisierung des Enols zum Keton das Cephalostatinanalogon der allgemeinen Formel Ic gebildet. Das Substitutionsmuster der Steroidringe dieses Hydroxyketons entspricht der im natürlich vorkommenden Cephalostatin 1 angetroffenen Situation.

Die Reduktion wird vorzugsweise mit Natrium- oder Tetrabutylammoniumboranat durchgeführt, und zwar in einer Lösung aus Dichlormethan und Methanol bei Temperaturen unterhalb -50 °C, beispielsweise bei -78 °C (Trockeneis/Aceton). Nach mehrstündigem Rühren wird die Reaktion durch Zugabe von Aceton zum Stillstand gebracht. Nach Aufarbeitung wird der Enolester nach üblichen Verfahren verseift, beispielsweise in Dichlormethanlösung mit 10 Äquivalenten Kaliumhydroxid, das in wenig Wasser gelöst wurde. Nach mehrstündigem Rückflußkochen wird die Reaktion durch Zugabe von Zitronensäure zum Stillstand gebracht. Nach Aufarbeitung und Chromatographie an Kieselgel wird das Cephalostatin-Analogon Ic in sehr guter Ausbeute (80%) erhalten.

Das nachfolgende Beispiel dient der näheren Erläuterung der Erfindung:

### BEISPIEL

### 2-Brom-(25R)-5α-spirost-14-en-3,12-dion (II)

500 mg (25R)-5α-spirost-14-en-3,12-dion werden in 15 ml Tetrahydrofuran gelöst und mit 453 mg (1,1 eq) Pyridiniumbromid-Perbromid, gelöst in 25 ml Tetrahydrofuran, versetzt. Nach zweieinhalbstündigem Rühren bei Raumtemperatur wird die Reaktion durch Zugabe von 20 ml Wasser die Reaktion gestoppt und das Gemisch mit Dichlormethan extrahiert. Die organische Phase wird mit Natriumchloridlösung gewaschen, über Magnesiumsulfat getrocknet, das Lösungsmittel entfernt und der Rückstand mit Petrolether/Ethylacetat (4:1) an Kieselgel chromatographiert. Die vereinigten Fraktionen werden eingedampft und über Nacht in 10 ml Methanol gerührt. Nach Filtration und Abdampfen des Lösungsmittels erhält man:
409 mg (70 %) α-Bromid und
111 mg (19 %) Gemisch aus α- und β-Bromid;
MS (FAB) α-Bromid (m/e): 507 (M⁺ + 2);
¹H-NMR (200 MHz, CDCl₃, δ): 5,48 tr,J=2 Hz, 15-H (1); 4,76 dd, J=2 Hz 8 Hz,16α-H (1); 4,72 dd, J=6 Hz 14Hz, 2β-H (1); 3,50 m, u.a. CH₂-27, (4); 1,32 s, CH₃-18 (3); 1,21 s, CH₃-19 (3); 1,05 d, J=7 Hz, CH₃-21 (3); 0,81 d, J=6 Hz, CH₃-26 (3).

### 2-Imino-(25R)-5α-spirost-14-en-3,12-dion (III)

500 mg (0,99 mmol) 2-Brom-(25R)-5α-spirost-14-en-3,12-dion (II) werden in 50 ml Dimethylformamid gelöst. Nach Zugabe von 700 mg Natriumazid und einiger Milligramm Natriumjodid wird die Lösung 2 Stunden lang bei 50 °C gerührt. Das Reaktionsgemisch wird auf Raumtemperatur gebracht, in 20 ml Wasser gegossen und mit Methyl-*tert.* -butylether extrahiert. Dieser Extrakt wird mit gesättigter Natriumchloridlösung gewaschen, über Magnesiumsulfat getrocknet und eingedampft. Man erhält so nach Kristallisation 407 mg (80 %) des Iminoketons III.
MS (m/e): 439 (M⁺);
¹H-NMR (200 MHz, CD₂Cl_{2,} δ): 5,86 s, =N-H (1); 5,41 tr, J=2 Hz, 15-H (1); 4,71 dd, J=2 Hz 8 Hz, 16α-H (1); 3,34 m, u.a. CH₂-27 (4); 1,30 s, CH₃-18 (3); 1,10 s, CH₃-19 (3); 1,01 d, J=7 Hz, CH₃-21 (3); 0,79 d, J=6 Hz, CH₃-26 (3).

### Bis[(25R)-5α-spirost-14-eno] [2,3-b;2',3'-e]pyrazin-12,12'-dion (IV)

897 mg (2,04 mmol) Iminoketon III werden in 50 ml Ethylacetat gelöst und mit 3 ml Methanol und 5 Tropfen Essigsäure versetzt. Nach Zugabe von 270 mg Palladium-Kohle (10 %) hydriert man bei Raumtemperatur (DC-Kontrolle). Nach Beendigung der Reaktion wird filtriert, das Filtrat mit gesättigter Natriumhydrogencarbonatlösung und gesättigter Natriumchloridlösung gewaschen, über Magnesiumsulfat getrocknet und eingedampft. Den Rückstand chromatographiert man mit Petrolether/Ethylacetat (2:1) an Kieselgel und erhält nach Kristallisation das Pyrazin IV in einer Ausbeute von 64 %.
MS(FAB, m/e): 845,5 (M⁺);
¹H-NMR (200 MHz, CDCH₃, δ): 5,49 s, br, (2); 4,78 dd, J=2 Hz 8 Hz (2); 3,44 m, u.a. CH₂-27 (6); 1,33 s, (6); 1,04 d, J=7 Hz, (6); 0,92 s, (6); 0,80 d, J=6 Hz (6).

### 12'-Pivaloyloxy-bis[(25R)-5α-spirost-14-eno][2,3-b;2',3'-e]pyrazin-11'en-12-on

### (Verbindung der allgemeinen Formel Ib)

100 mg Pyrazindiketon IV löst man unter Argon in 7 ml absolutem Tetrahydrofuran und fügt dann bei Raumtemperatur 3,2 Äquivalente Kaliumhexamethyldisilazan hinzu. Nach 30 Minuten versetzt man mit 0,16 ml Pivaloylchlorid und rührt 15 Stunden bei Raumtemperatur. Die Lösung wird dann in kalte wäßrige Zitronensäure gegossen und mit Methyl-*tert*.-butylether extrahiert. Der Extrakt wird mit gesättigter Natriumhydrogencarbonatlösung und mit gesättigter Natriumchloridlösung gewaschen, über Magnesiumsulfat getrocknet, eingedampft und an Kieselgel chromatographiert. Man gewinnt auf diese Weise 44 mg (40 %) des Monopivalates Ib.
MS(FAB, m/e): 929,7 (M⁺);
¹H-NMR (200 MHz, CDCl₃, δ): 5,49 m, (2); 5,21 s, br., (1); 4,81 dddd, J=2 Hz 8 Hz (2); 3,45 m, u.a. CH₂-27,27' (6); 1,33 s (3); 1,28 s (9); 1,22 s, (3); 1,05 dd, J=7 Hz (6); 0,92 s (3); 0,89 s (3); 0,80 d, J=6 Hz (6).

### 12β-Hydroxy-bis[(25R)-5α-spirost-14-eno] [2,3-b;2',3'-e]pyrazin-12'-on (Ic)

(Reduktion des Monoenolesters Ib und Freisetzung der 12-Keto-12'-hydroxy-Verbindung Ic) 63 mg (0,0678 mmol) Monopivalat Ib werden in 8 ml Solvens (Dichlormethan/Methanol 1:1) gelöst und bei -78 °C mit 5 mg (4 eq) Natriumboranat versetzt. Nach 3 Stunden Rühren bei -78 °C wird mit 1,5 ml Aceton gequencht, das Gemisch auf Raumtemperatur gebracht, mit Dichlormethan verdünnt, mit 1 M NaOH-Lösung gewaschen, über Magnesiumsulfat getrocknet, das Solvens entfernt und der Rückstand an Kieselgel chromatographiert (Petrolether/Ethylacetat 1:1). Überprüfung des Reaktionsproduktes erfolgt anhand des IR-Spektrums. Die nachfolgende Verseifung des Enolesters geschieht in Dichlormethan/ Methanol (1:1) mit 10 eq KOH, gelöst in wenig Wasser. Nach 6 Stunden Rückflußkochen wird auf Raumtemperatur abgekühlt, mit verdünnter Zitronensäure gequencht und das Gemisch mit Dichlormethan extrahiert. Nach Waschen des organischen Extrakts mit verdünnter Natriumhydrogencarbonat- und Natriumchloridlösung wird über Magnesiumsulfat getrocknet, das Solvens entfernt und der Rückstand an Kieselgel chromatographiert (Petrolether/Ethylacetat 1:1). Ausbeute bezogen auf Ib: 78 %.
MS (FAB, m/e): 847,6 (M⁺);
¹H-NMR(200 MHz, CD₂Cl_{2,} δ): 5,42 tr, J< 1 Hz (1); 5,37 tr, J < 1Hz (1); 4,83 dd, J=2 Hz 8 Hz (1); 4,73 dd, J=2 Hz 8 Hz (1); 3,36 m, (6); 1,31 s (3); 1,01 d, J=7 Hz (6); 1,01 s (3); 0,90 s (3); 0,85 s (3); 0,80 d, J=6 Hz (6).

### Bis[(25R)-5α-spirost-14-eno][2,3-b;2',3'-e]pyrazin-12β,12β'-diol (Id)

110 mg des Pyrazindiketons IV (0,13mmol) werden in ca. 10 ml Dichlormethan gelöst und mit 134 mg (4 eq = 0,5206 mmol) Tetrabutylammoniumborhydrid versetzt und 4 Stunden lang bei Raumtemperatur gerührt. Zum Abbruch der Reaktion wird die Reaktionsmischung mit 2 N Zitronensäurelösung versetzt. Die wäßrige Phase wird abgetrennt und mehrmals mit Dichlormethan extrahiert. Die vereinigten organischen Phasen werden mit verdünnter Natriumhydrogencarbonatlösung und gesättigter Natriumchloridlösung gewaschen, über Magnesiumsulfat getrocknet und am Rotationsverdampfer eingeengt. Zur Reinigung wird über Kieselgel mit Petrolether/Ethylacetat (1:1) chromatographiert. Man erhält 56 mg (0,0659 mmol = 50,6 % Ausbeute) des Diols Id.

## Patentansprüche

1. Cephalostatin-Analoga der allgemeinen Formel I, 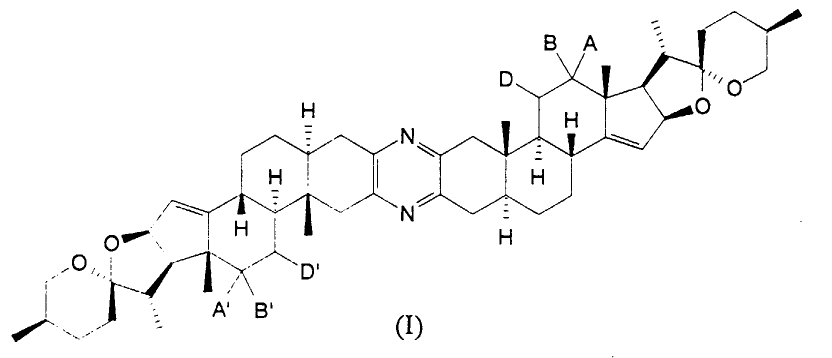 worin
a) A und A' je für eine Alkanoyloxygruppe R-CO-O- mit R als gerad- oder verzweigtket tigen C1- bis C7-Alkylrest und B und D sowie B' und D' je gemeinsam für eine zusätzliche C-C-Bindung oder
b) A' für eine Alkanoyloxygruppe R-CO-O- mit R als gerad- oder verzweigtkettigen C₁- bis C₇-Alkylrest und B' und D' gemeinsam für eine zusätzliche C-C-Bindung sowie A und B gemeinsam für ein Ketosauerstoffatom und D für ein Wasserstoffatom oder
c) A für eine β-ständige Hydroxygruppe und B sowie D je für ein Wasserstoffatom sowie A' und B' gemeinsam für ein Ketosauerstoffatom und D' für ein Wasserstoffatom oder
d) A und A' je für eine β-ständige Hydroxygruppe und B, D, B' und D' je für ein Wasserstoffatom
stehen.

2. 12β-Hydroxy-bis[(25R)-5α-spirost-14-eno][2,3-b;2',3'-e]pyrazin-12'-on und Bis[(25R)-5α-spirost-14-eno][2,3-b;2',3'-e]pyrazin-12β,12β'-diol gemäß Anspruch 1.

3. Verfahren zur Herstellung der Cephalostatin-Analoga der allgemeinen Formel I gemäß Anspruch 1 oder 2, dadurch gekennzeichnet, daß die Verbindung der Formel II, 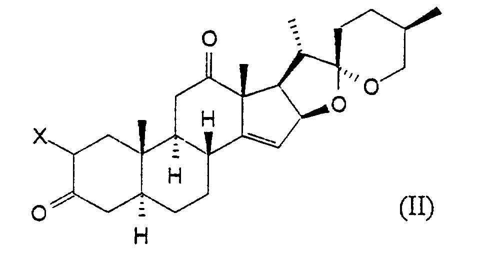 worin X für ein Bromatom steht, durch Umsetzung mit einem Alkaliazid AlkaliN₃, worin Alkali für ein Lithium-, Natrium- oder Kaliumatom steht, in die Verbindung der Formel II, worin X für die Azidgruppe -N₃ steht, überführt,
dieses α-Ketoazid unter Erwärmen und Stickstoffverlust zum 2-Imino-3-keton der Formel III 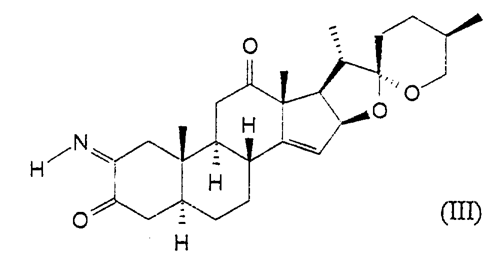 abgebaut,
dieses unter Bedingungen einer katalytischen Hydrierung zum symmetrischen Pyrazindiketon der Formel IV 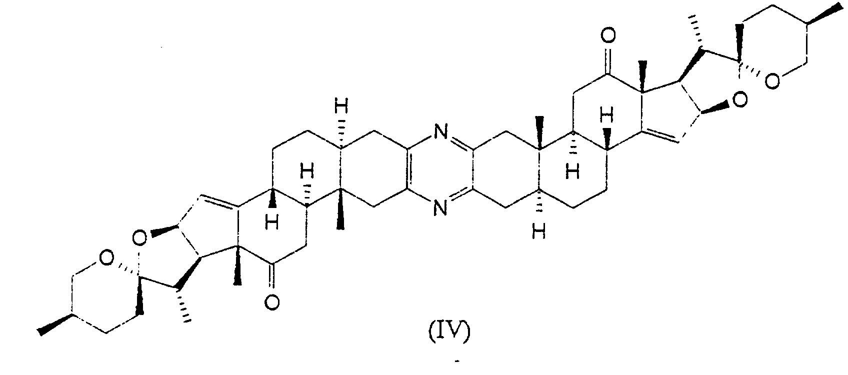 dimerisiert, und, wenn eine Verbindung der allgemeinen Formel I mit der Substituentendefinition a), b) oder d) hergestellt werden soll,
a) dieses Bis-12,12'-keto-steroidopyrazin durch Überführung beider Ketogruppen in das Enolat und dessen Abfang als C₂- bis C₈-Carbonsäureester mit einem Carbonsäurechlorid oder -bromid oder einem Carbonsäureanhydrid der allgemeinen Formel V,
R-CO-Y bzw. (R-CO)₂O (V)
worin
R einen gerad- oder verzweigtkettigen C₁- bis C₇-Alkylrest und
Y ein Chlor- oder Bromatom
darstellen, in einen Diester der allgemeinen Formel Ia, 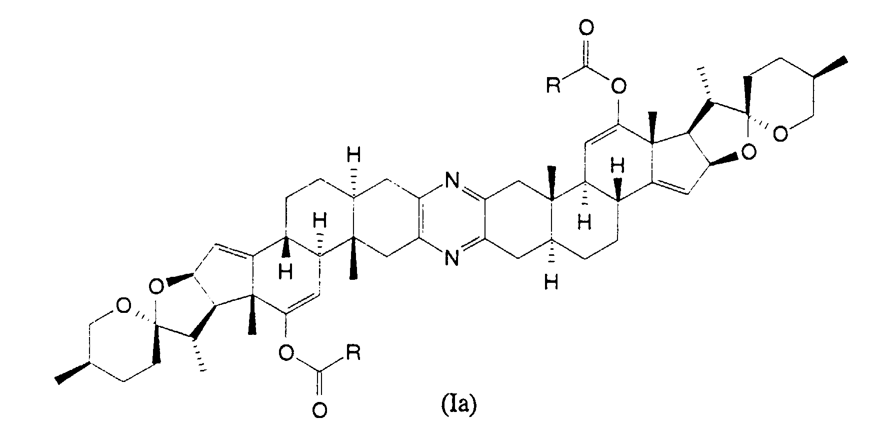 worin R einen gerad- oder verzweigtkettigen C₁-C₇-Alkylrest bedeutet,
überführt oder
b) dieses Bis-12,12'-keto-steroidopyrazin durch Überführung einer der Ketogruppen in das Enolat und dessen Abfang mit einer Verbindung der allgemeinen Formel V als C₂- bis C₈-Carbonsäureester zu einem Monoester der allgemeinen Formel Ib, 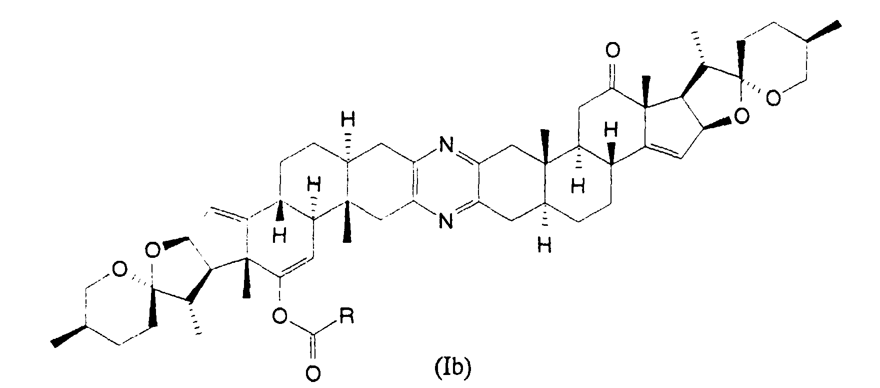 worin R einen gerad- oder verzweigtkettigen C₁- bis C₇-Alkylrest bedeutet,
umgesetzt, und gegebenenfalls
c) aus der 12-Keto-12'-alkanoyloxy-Verbindung der allgemeinen Formel Ib durch Reduk tion der 12-Ketogruppe mit einem Alkali- oder Ammoniumborhydrid (Alkali = Lithium, Natrium, Kalium; Ammonium = NH₄⁺ oder NAlkyl₄⁺, Alkyl = C₁-C₄) und Verseifung (und Tautomerisierung) der 12-Estergruppe in die Verbindung der Formel Ic 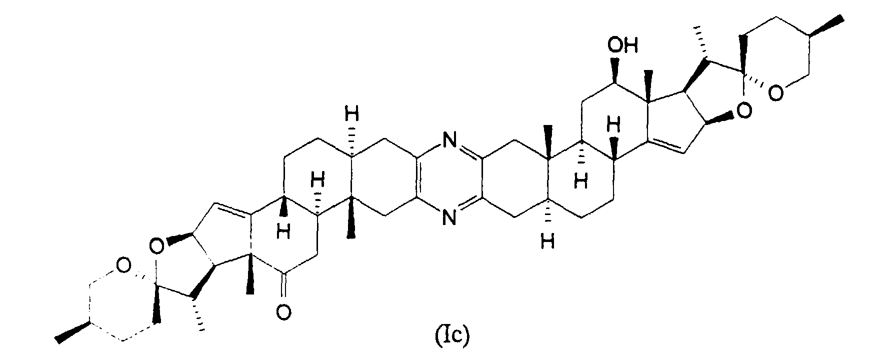 umgewandelt oder
d) dieses Bis-12,12'-keto-steroidopyrazin mit einem Alkali- oder Ammoniumborhydrid (Alkali und Ammonium vgl. Ic) zum Bis-12,12'-hydroxy-steroidopyrazin der allgemeinen Formel Id 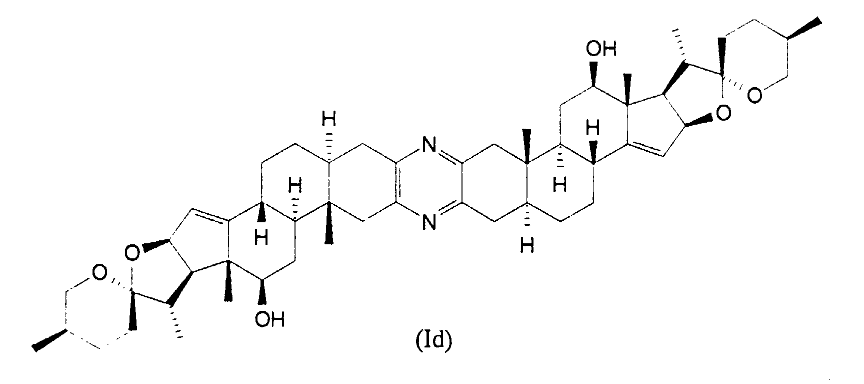 reduziert wird.

4. Zwischenprodukt der Formel IV

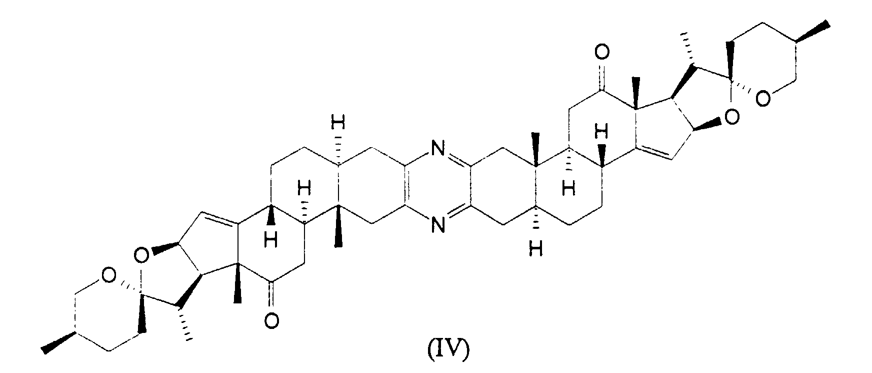

5. Pharmazeutische Präparate enthaltend mindestens ein Cephalostatin-Analogon der allgemeinen Formel I gemäß Anspruch 1 oder 2 sowie einen pharmazeutisch verträglichen Träger.

6. Verwendung der Cephalostatin-Analoga der allgemeinen Formel I gemäß Anspruch 1 oder 2 zur Herstellung von Arzneimitteln.

## Claims

1. Cephalostatin analogues of general formula I, 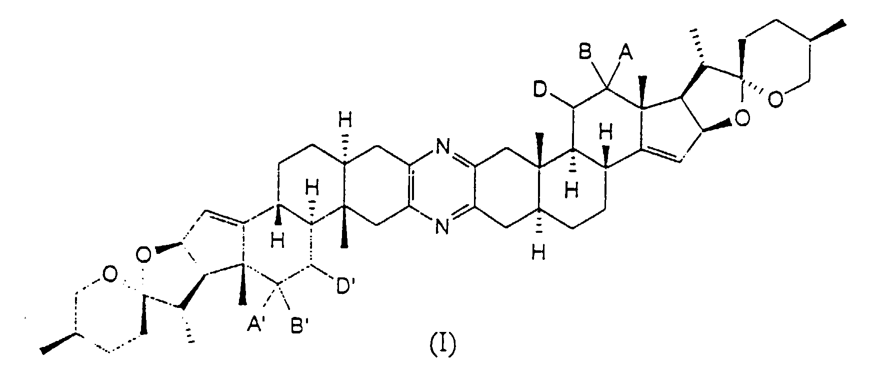 in which
a) A and A' each stand for an alkanoyloxy group R-CO-O- with R as a straight-chain or branched-chain C₁ to C₇ alkyl radical, and B and D as well as B' and D' each together stand for an additional C-C bond, or
b) A' stands for an alkanoyloxy group R-CO-O- with R as a straight-chain or branched-chain C₁ to C₇ alkyl radical, and B' and D' together stand for an additional C-C bond, and A and B together stand for a keto-oxygen atom and D stands for a hydrogen atom, or
c) A stands for a β-position hydroxy group, and B as well as D each stand for a hydrogen atom, and A' and B' together stand for a keto-oxygen atom, and D' stands for a hydrogen atom or
d) A and A' each stand for a β-position hydroxy group, and B, D, B' and D' each stand for a hydrogen atom.

2. 12β-Hydroxy-bis[(25R)-5α-spirost-14-eno][2,3-b;2',3'-e]pyrazin-12'-one and
bis[(25R)-5α-spirost-14-eno][2,3-b[2',3'-e]pyrazine-12β,12β'-diol according to claim 1

3. Process for the production of cephalostatin analogues of general formula I according to claim 1 or 2, characterized in that the compound of formula II, 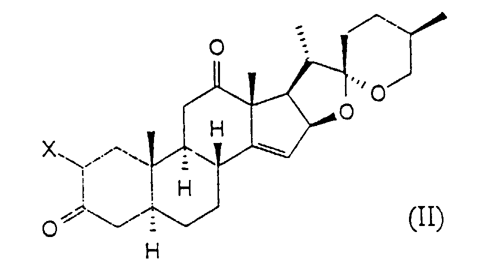 in which X stands for a bromine atom, is converted by reaction with an alkali azide AlkN₃, in which Alk stands for a lithium, sodium or potassium atom, to the compound of formula II, in which X stands for the azide group -N₃,
this α-ketoazide is catabolized with heating and nitrogen loss to form 2-imino-3-ketone of formula III 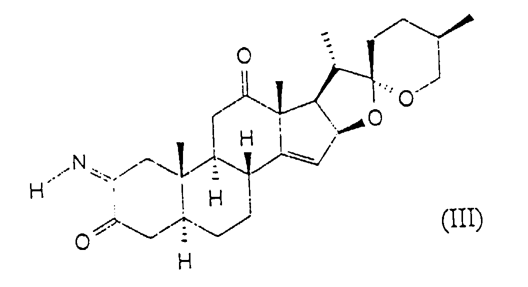 the latter is dimerized under conditions of catalytic hydrogenation to form symmetrical pyrazinediketone of formula IV, 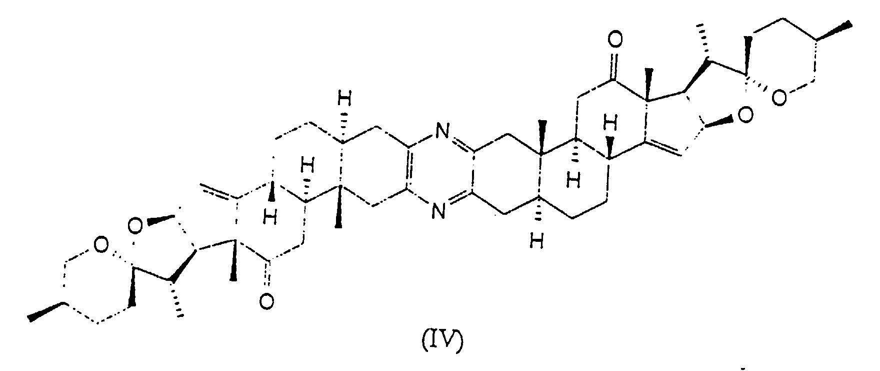 and, if a compound of general formula I is to be produced with substituent definition a), b) or d),
a) this bis-12,12'-keto-steroidopyrazine is converted by conversion of both keto groups to the enolate and its trapping as a C₂ to C₈ carboxylic acid ester with a carboxylic acid chloride or -bromide or a carboxylic anhydride of general formula V,
R-CO-Y or (R-CO)₂O (V)
in which
R represents a straight-chain or branched-chain C₁ to C₇ alkyl radical and
Y represents a chlorine or bromine atom,
to form a diester of general formula Ia, 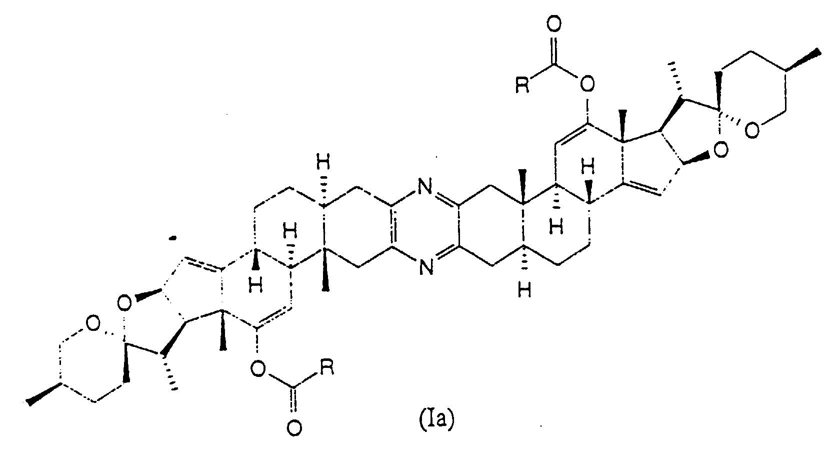 in which R means a straight-chain or branched-chain C₁-C₇ alkyl radical, or
b) this bis-12,12'-keto-steroidopyrazine is reacted by conversion of one of the keto groups in the enolate and its trapping with a compound of general formula V as a C₂ to C₈ carboxylic acid ester being reacted to form a monoester of general formula Ib, 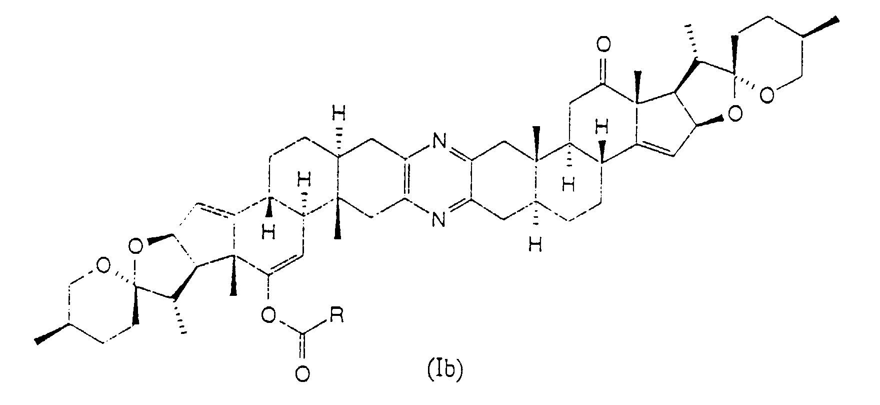 in which R means a straight-chain or branched-chain C₁ to C₇ alkyl radical, and optionally
c) is converted from the 12-keto-12'-alkanoyloxy compound of general formula Ib by reduction of the 12-keto group with an alkali or ammonium borohydride (alkali = lithium, sodium, potassium; ammonium = NH₄⁺ or NAlkyl₄⁺, alkyl = C₁-C₄) and saponification (and tautomerization) of the 12'-ester group to form the compound of formula Ic 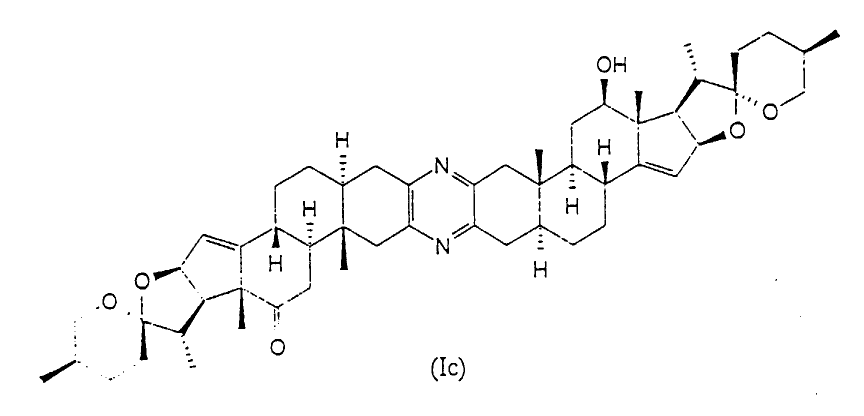 or
d) this bis-12,12'-keto-steroidopyrazine is reduced with an alkali or ammonium borohydride (alkali and ammonium cf. Ic) to form bis-12,12'-hydroxy-steroidopyrazine of general formula Id.

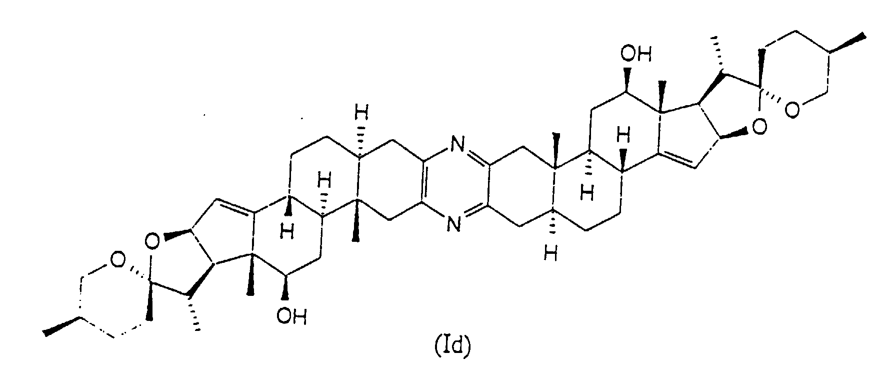

4. Intermediate product of formula IV

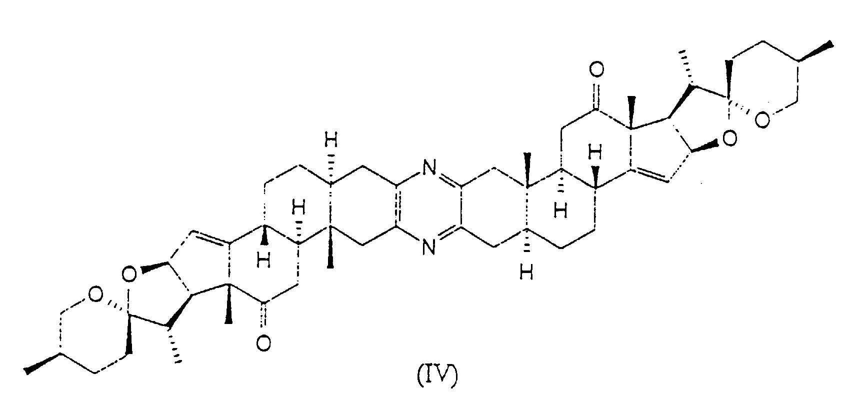

5. Pharmaceutical preparations that contain at least one cephalostatin analogue of general formula I according to claim 1 or 2 and a pharmaceutically compatible vehicle.

6. Use of the cephalostatin analogues of general formula I according to claim 1 or 2 for the production of pharmaceutical agents.

## Revendications

1. Analogues de la céphalostatine de formule générale I : 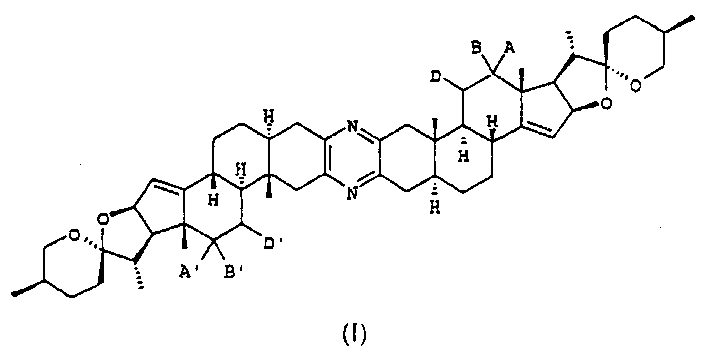 dans laquelle :
a) A et A' représentent chacun un groupe alcanoyloxy R-CO-O, R étant un reste alkyle en C₁ à C₇ à chaîne droite ou ramifiée, et B et D ensemble ainsi que B' et D' ensemble représentent chacun une liaison C-C supplémentaire, ou bien
b) A' représente un groupe alcanoyloxy R-CO-O, R étant un reste alkyle en C₁ à C₇ à chaîne droite ou ramifiée, et B' et D' ensemble représentent une liaison C-C supplémentaire et A et B ensemble représentent un atome d'oxygène cétonique et D représente un atome d'hydrogène, ou bien
c) A représente un groupe hydroxy en position β et B ainsi que D représentent chacun un atome d'hydrogène, et A' et B' ensemble représentent un atome d'oxygène cétonique et D' représente un atome d'hydrogène, ou bien
d) A et A' représentent chacun un groupe hydroxy en position β et B, D, B' et D' représentent chacun un atome d'hydrogène.

2. 12β-hydroxy-bis[(25R)-5α-spirost-14-éno][2,3-b;2',3'-e] pyrazin-12'-one et bis[(25R)-5α-spirost-14-éno][2,3-b;2',3'-e] pyrazin-12β-12β'-diol selon la revendication 1.

3. Procédé de préparation d'analogues de la céphalostatine de formule générale I selon la revendication 1 ou 2, caractérisé en ce que l'on convertit le composé de formule II : 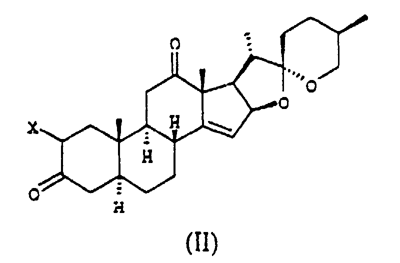 dans laquelle X représente un atome de brome, en un composé de formule Il, dans laquelle X représente le groupe azide -N₃, en le faisant réagir avec un azide alcalin [alcali]N₃, [alcali] représentant un atome de lithium, de sodium ou de potassium ;
en ce que l'on décompose cet azide α-cétonique en le chauffant et avec perte d'azote en une 2-imino-3-cétone de formule III: 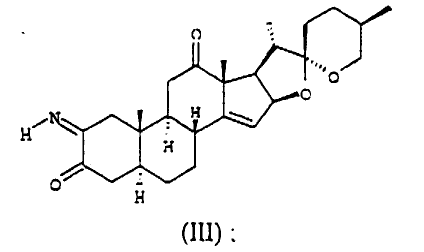 en ce que l'on dimérise cette dernière dans des conditions d'hydrogénation catalytique pour obtenir une pyrazine dicétone symétrique de formule IV: 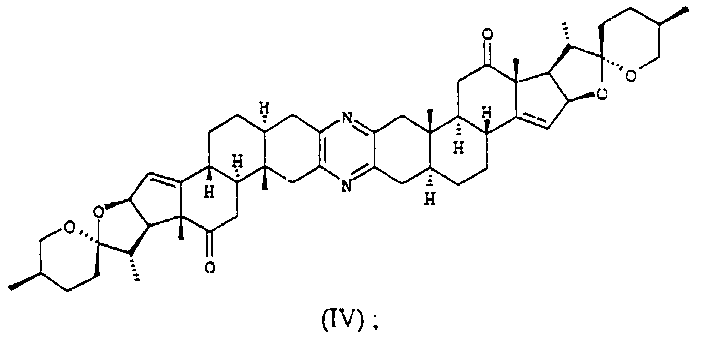 et en ce que, lorsque doit être préparé un composé de formule générale I avec des substituants répondant aux définitions a), b) ou d) :
a) on convertit cette bis-12,12'-cétostéroïdopyrazine, par transformation des deux groupes cétoniques en énolate et traitement de ce dernier, pour former un carboxylate en C₂ à C₈ , au moyen d'un chlorure ou d'un bromure carboxylique ou bien d'un anhydride carboxylique de formule générale V :
R-CO-Y ou (R-CO)₂O (V)
dans laquelle :
R représente un reste alkyle en C₁ à C₇ à chaîne droite ou ramifiée et
Y représente un atome de chlore ou de brome,
en un diester de formule générale la : 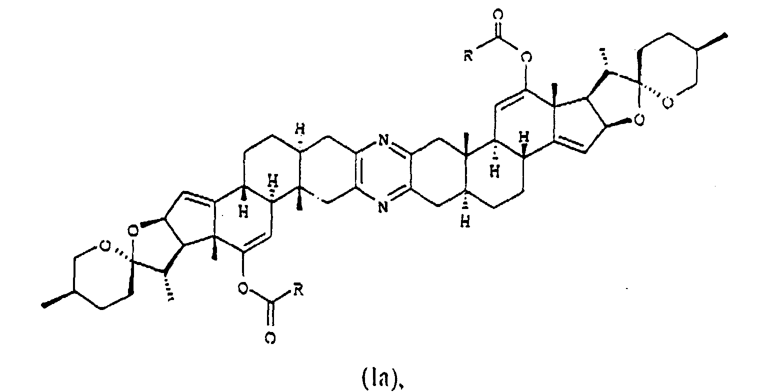 dans laquelle R représente un reste alkyle en C₁ à C₇ à chaîne droite ou ramifiée, ou bien
b) on fait réagir cette bis-12,12'-cétostéroïdopyrazine, par transformation de l'un des groupes cétoniques en énolate et traitement de ce dernier, pour former un carboxylate en C₂ à C₈, avec un composé de formule générale V pour obtenir un monoester de formule générale Ib : 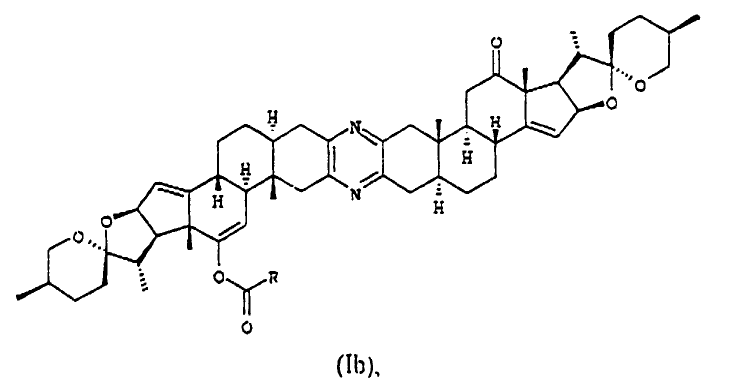 dans laquelle R représente un reste alkyle en C₁ à C₇ à chaîne droite ou ramifiée, et le cas échéant
c) on convertit le composé 12-céto-12'-alcanoyloxy de formule générale Ib, par réduction du groupe 12-céto avec un borohydrure de métal alcalin ou d'ammonium (métal alcalin = lithium, sodium, potassium, ammonium = NH₄⁺ ou N(alkyle)₄', alkyle = C₁ à C₄) et saponification (et tautomérisation) du groupe 12-ester, en un composé de formule Ic : 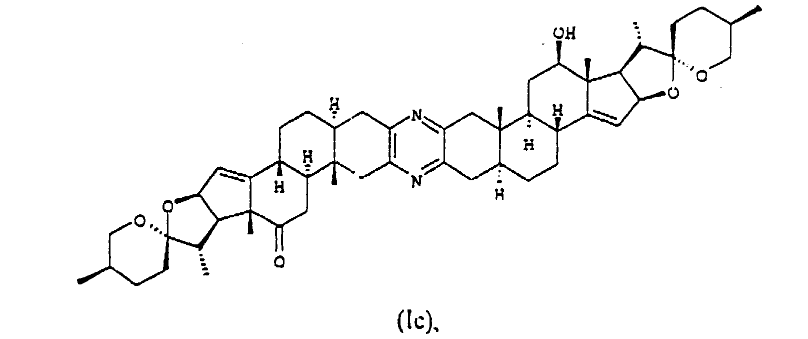 ou bien
d) on réduit cette bis-12,12'-cétostéroïdopyrazine au moyen d'un borohydrure de métal alcalin ou d'ammonium (métal alcalin et ammonium : voir Ic) en une bis-12,12'-hydroxy-stéroïdopyrazine de formule générale Id:

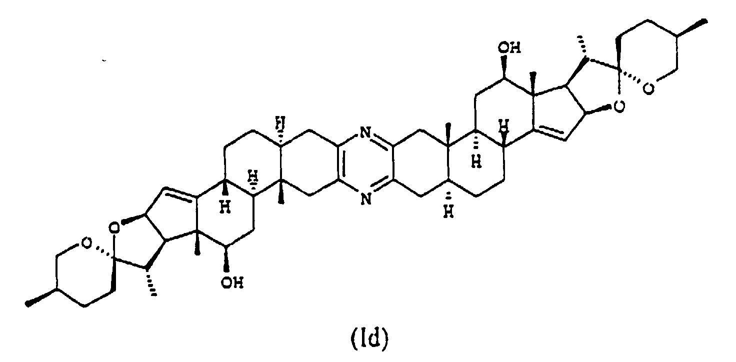

4. Produit intermédiaire de formule IV :

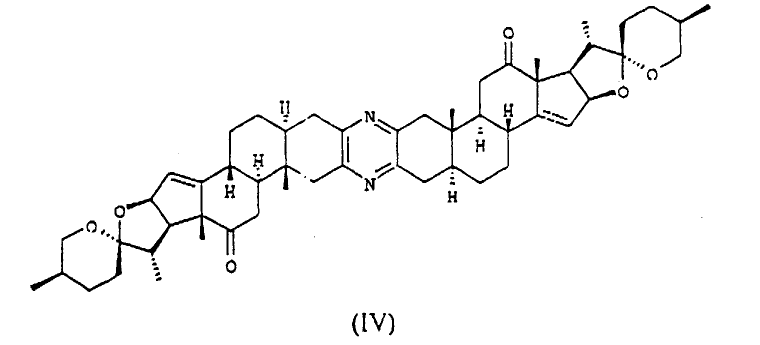

5. Préparations pharmaceutiques contenant au moins un analogue de la céphalostatine de formule générale I selon la revendication 1 ou 2 ainsi qu'un support pharmaceutiquement compatible.

6. Utilisation des analogues de la céphalostatine de formule générale I selon la revendication 1 ou 2 pour fabriquer des médicaments.
